# EUROPEAN PATENT SPECIFICATION

(11) **EP 1 478 611 B1**
(45) Date of publication and mention of the grant of the patent: **02.06.2010**
(21) Application number: 02707892.2
(22) Date of filing: 28.02.2002
(51) Int. Cl.: C07C 2/58

(54) **SOLID CATALYST ALKYLATION PROCESS WITH REGENERATION SECTION AND HYDROGEN FRACTIONATION ZONE**
ALKYLIERUNGSVERFAHREN, IN DEM EIN FESTER KATALYSATOR VERWENDET WIRD UND DAS EINE REGENERIERUNGSZONE UND EINE WASSERSTOFFFRAKTIONIERUNGSZONE UMFASST
PROCEDE D'ALKYLATION A CATALYSEUR SOLIDE FAISANT INTERVENIR UNE SECTION DE REGENERATION ET UNE ZONE DE FRACTIONNEMENT D'HYDROGENE

(43) Date of publication of application: 24.11.2004
(73) Proprietor: UOP LLC, Des Plaines, IL 60017 (US)
(72) Inventor: SHIELDS, Dale James, Buffalo Grove, IL 60089 (US); SECHRIST, Paul Alvin, Des Plaines, IL 60016 (US)
(74) Representative: Hayes, Adrian Chetwynd
(86) International application number: PCT/US2002/005738
(87) International publication number: WO 2003/074451

(56) References cited:
- US-A- 5 672 798

## Description

### BACKGROUND OF THE INVENTION

This invention relates to a process for producing motor fuel blending components by alkylating paraffins with olefins using a solid catalyst, which is regenerated in the presence of hydrogen.

Hydrocarbon alkylation is widely used in the petroleum refining and petrochemical industries to produce a variety of useful acyclic and cyclic hydrocarbon products that are consumed in motor fuel, plastics, detergent precursors, and petrochemical feedstocks. Alkylation comprises reacting an alkylation substrate feedstock such as isobutane and benzene with an alkylation agent feedstock such as C₂-C₂₂ olefins. For example, large amounts of paraffins for high-octane gasoline are produced by the alkylation of isobutane with butenes. In addition, valuable aromatic hydrocarbons including cumene, ethylbenzene, and C₁₆-C₂₂ linear alkylaromatics are produced in large amounts by alkylating benzene with olefins of the appropriate carbon number. Much of the installed base of alkylation capacity uses liquid phase hydrofluoric acid, generally referred to as HF, as the catalyst.

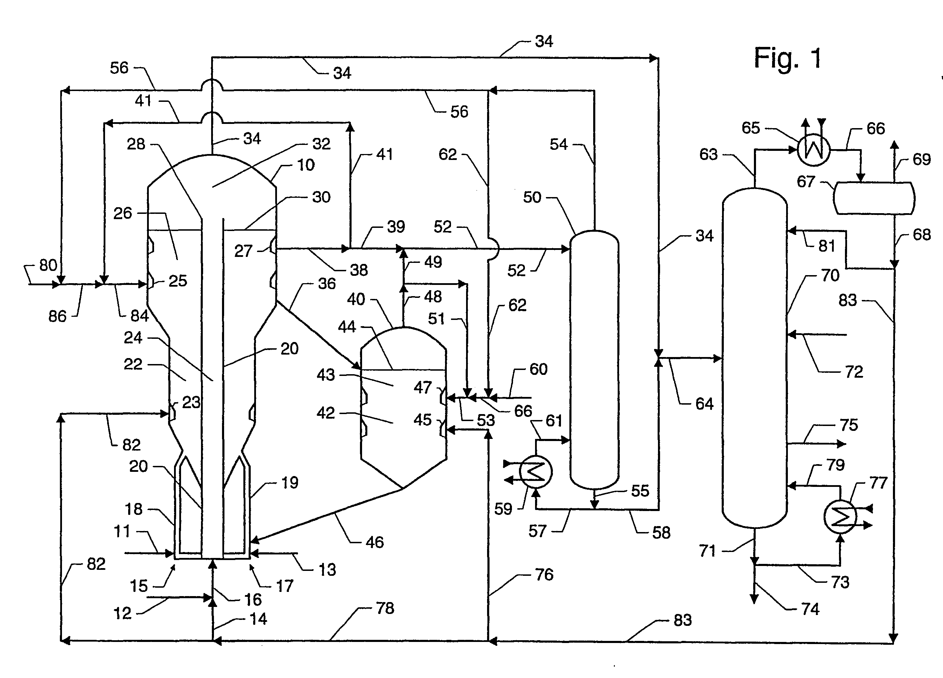

Figures 1.4.3 and 1.4.4 of the book entitled Handbook of Petroleum Refining Processes, edited by Robert A. Meyers, Second Edition, McGraw-Hill, New York, 1997, show process flow diagrams of HF alkylation processes, including the product recovery facilities for recovering the hydrocarbons in the alkylation reactor effluent. The use of HF in these motor fuel and detergent processes has a long record of highly dependable and safe operation. The need to safely dispose of some byproducts produced in the process has led to an increasing demand for alkylation process technology which does not employ liquid phase HF as the catalyst. US-A- 5,672,798, for example, discloses alkylating paraffinic hydrocarbons such as isobutane with olefinic hydrocarbons such as propylene or butenes in a fluidized riser-reactor using a solid catalyst. The effluent of the riser-reactor contains the desired alkylate product. The solid catalyst is separated and the remainder of the riser-reactor effluent passes to product recovery facilities.

US-A- 5,672,798 teaches a number of suitable solid catalysts that contain or have been treated with a Lewis acid, such as a large pore zeolite and a Lewis acid such as boron trifluoride and aluminum chloride, a large pore crystalline molecular sieve and a gaseous Lewis acid, a crystalline transition alumina treated with a Lewis acid, an acid washed silica treated with antimony pentafluroride, and a refractory inorganic oxide impregnated with a monovalent cation whose bound surface hydroxyl groups have been at least partially reacted with a Friedel-Crafts metal fluoride, chloride, or bromide. These catalysts appear to suffer from slight but significant halogen loss rates when used at commercially useful alkylation reactor conditions. The gradual depletion of halogen results in a change in product composition and also requires the occasional replenishing of the halogen content of the catalyst. Some of the halogen loss is believed to be caused by the stripping of halogen from catalytically active sites of the catalyst by isobutane and also by the deposition on the catalytically active sites of heavy compounds. As used herein, the term "heavy compounds" means molecules that have at least one carbon atom more than the number of carbon atoms than the highest number of carbon atoms of those molecules that are desired to be in the alkylate.

In addition to exhibiting halogen loss, these catalysts also seem to suffer from unacceptably high deactivation rates when employed at commercially feasible conditions. The rapid change in activity results in a change in product composition and requires the periodic regeneration of the catalyst. Such periodic regeneration is typically accomplished by removing deactivated catalyst from the reaction zone, reactivating the catalyst in a separate zone, and returning the reactivated catalyst to the reaction zone. Some of the deactivation is believed to be caused by the deposition of heavy compounds on the catalytically active sites of the catalyst. US-A- 5,672,798 describes removing the heavy hydrocarbon deposits and at least partially restoring the activity of the catalyst by contacting the catalyst within the process with hydrogen in two separate and simultaneous modes of regeneration: a mild liquid-phase washing and a hot vapor-phase stripping. The hot vapor-phase stripping consists of contacting the catalyst with a vapor-phase gas stream at a temperature that is typically greater than that employed in the alkylation zone. Because the gas stream uses hydrogen and the contacting occurs at an elevated temperature, hot vapor-phase stripping, which is also referred to as "hydrogen stripping" or "severe regeneration." US-A- 5,627,798 also teaches that some isobutane in the gas stream desirably increases the heat capacity of the gas and thereby increases the catalyst heat-up rates. This hot hydrogen-isobutane stripping removes liquid phase hydrocarbons and deposits of heavy compounds from the catalyst and produces a vapor phase regeneration zone effluent stream that is condensed and separated to pass the recovered liquids to products recovery facilities, and recycle hydrogen to the severe regeneration zone.

US-A- 5,310,713 and 5,672,798 disclose washing the catalyst in mild liquid-phase, preferably with the feed alkylation substrate (e.g., isobutane). This washing generally occurs at a lower temperature than severe regeneration, and is often referred to as "mild regeneration." US-A- 5,310,713 and 5,672,798 teach hydrogen dissolution up to the stoichiometrically required amount in this liquid-phase stream by a controlled addition of hydrogen. For purposes of computing the stoichiometric requirement, these patents teach laboratory analysis of catalyst for its heavy hydrocarbon deposits while assuming the heavy hydrocarbon deposits comprise monoolefinic octenes. Some of this hydrogen is chemically consumed by saturating unsaturated hydrocarbons on the catalyst surface. The mild regeneration provides reactivated catalyst and a liquid-phase effluent. This mild regeneration effluent usually contains hydrogen up to the point of saturation of hydrogen.

The severe or mild regeneration zones typically contain hydrogen in excess of the amount that reacts with heavy hydrocarbon deposits in that zone. Hydrogen in the effluent(s) of these zones can still be useful in regenerating the catalyst. Therefore, methods are sought to recover and recycle hydrogen that is present in the regeneration effluent(s).

### SUMMARY OF THE INVENTION

This invention is a paraffin-olefin alkylation process using a solid catalyst with a catalyst regeneration zone, in which an alkylation reactor effluent passes to an alkylate fractionation zone and a hydrogen-containing regeneration effluent passes to a hydrogen fractionation zone. The alkylate fractionation zone recycles unreacted paraffinic feed or halogen-containing species to the alkylation reactor to maintain the halogen content of the catalyst in the alkylation reactor. At the same time, the hydrogen fractionation zone recycles molecular hydrogen to the regeneration zone to reactivate the catalyst. The hydrogen fractionation zone keeps molecular hydrogen from mixing with the reactor effluent and from entering the alkylate fractionation zone, thereby preventing hydrogen recycle to the alkylation reactor. By segregating molecular hydrogen in the regeneration effluent from the reactor effluent, the alkylate fractionation zone can in one embodiment of this invention produce a recycle stream comprising unreacted paraffinic feed or halogen-containing species that is substantially free of molecular hydrogen, that is, less than 500 wt-ppm molecular hydrogen. Therefore, the hydrogen fractionation zone maximizes the use of molecular hydrogen for regeneration and minimizes passing of molecular hydrogen to the alkylation reactor.

US-A- 5,672,798 does not pass either the mild regeneration effluent or the severe regeneration effluent to a hydrogen fractionation zone, and therefore uses the olefin alkylating agent very inefficiently. US-A- 5,672,798 teaches combining the mild regeneration effluent with the riser-reactor effluent and passing the combined effluents to the product recovery facilities and inevitably mixing the hydrogen chloride in the riser-reactor effluent with the molecular hydrogen in the regeneration effluent. Relatively close volatilities of molecular hydrogen and hydrogen chloride at commercially feasible fractionation make molecular hydrogen and hydrogen chloride difficult to separate in the isostripper, thus the isostripper overhead stream typically contains both molecular hydrogen and hydrogen chloride. Therefore, recycling of the overhead stream to the inlet of the riser-reactor in order to replenish the chloride content of the catalyst would also recycle molecular hydrogen to the inlet of the riser-reactor. Introducing molecular hydrogen at a point where unreacted olefin is present degrades alkylation performance by allowing molecular hydrogen to saturate the olefin and thereby renders the olefin ineffective as an alkylating agent. In contrast, this invention prevents the entrance of molecular hydrogen into the alkylate fractionation zone, and recovers and recycles molecular hydrogen in the mild and/or severe regeneration effluents to effectively use the alkylating agent. In the case of the severe regeneration effluent, US-A- No. 5,672,798 teaches passing the severe regeneration effluent to a vapor-liquid separator, separating a heavy hydrocarbon liquid phase from the vapor phase, and passing the liquid phase to conventional product recovery facilities. This invention recognizes, however, that a significant portion of the molecular hydrogen that enters the vapor-liquid separator with the severe regeneration effluent exits the vapor-liquid separator with the liquid phase, rather than the vapor phase, and eliminates the passage of significant and unacceptable quantities of molecular hydrogen to the isostripper and in turn to the riser-reactor.

This invention also reduces the capital cost and operating costs of the isostripper by not only preventing the mixture of molecular hydrogen and hydrogen chloride but also that of molecular hydrogen and the alkylation substrate (e.g., isobutane), in the isostripper. The typical introduction of isobutane in stoichiometric excess usually brings isobutane into the alkylation reaction effluent, so that the isostripper recycles isobutane to the alkylation reaction zone. Separating molecular hydrogen that enters the isostripper from hydrogen chloride and the isobutane requires a significant increase in the number of isostripper trays, especially in its upper section, as well as a significant increase in the reboiler duty. By using a hydrogen fractionation zone, this invention avoids the cost of adding these additional trays and providing additional heat utilities.

A broad objective of this invention is to alkylate paraffins with olefins using a regenerable solid catalyst in which hydrogen is used efficiently for regeneration while avoiding any detrimental reaction of hydrogen and olefins. This invention is well-suited for processes that use a solid catalyst and in which a halogen maintains catalyst performance because this invention can recycle halogens in order to replenish the catalyst halogen content.

Accordingly, in a broad embodiment, this invention is an alkylation process comprising passing a first feed stream comprising a paraffinic alkylation substrate and a second feed stream comprising an olefinic alkylating agent to an alkylation reaction zone. The alkylation reaction zone operates at alkylation conditions selected to react the paraffinic alkylation substrate and the olefinic alkylating agent in the presence of a solid catalyst to produce alkylate. The alkylation conditions are also sufficient to deposit heavy compounds on the solid catalyst in the alkylation reaction zone. An alkylation reaction effluent comprising the alkylate and the paraffinic alkylation substrate is withdrawn from the alkylation reaction zone. A first catalyst stream comprising solid catalyst having heavy compounds deposited thereon is withdrawn from the alkylation reaction zone. At least a portion of the first catalyst stream passes to a first regeneration zone. The solid catalyst having heavy compounds deposited thereon is contacted with molecular hydrogen in the first regeneration zone at first regeneration conditions selected to remove at least a portion of the heavy compounds from the solid catalyst having heavy compounds deposited thereon and to at least partially regenerate the solid catalyst having heavy compounds deposited thereon. A second catalyst stream comprising at least partially regenerated solid catalyst is withdrawn from the first regeneration zone. At least a portion of the second catalyst stream passes to the alkylation reaction zone. A first regeneration effluent comprising molecular hydrogen and the heavy compounds is withdrawn from the first regeneration zone. At least a portion of the first regeneration effluent passes to a hydrogen fractionation zone. A hydrogen-enriched stream having a first concentration of molecular hydrogen is recovered from the hydrogen fractionation zone. A hydrogen-depleted stream comprising the heavy compounds and having a second concentration of molecular hydrogen that is less than the first concentration of molecular hydrogen is also recovered from the hydrogen fractionation zone. At least a portion of the hydrogen-enriched stream passes to the first regeneration zone. At least a portion of the alkylation reaction effluent and at least a portion of the hydrogen-depleted stream passes to an alkylate fractionation zone. A recycle stream comprising the paraffinic alkylation substrate is withdrawn from the alkylate fractionation zone. The first feed stream is formed from at least a portion of the recycle stream. The alkylate is recovered from the alkylate fractionation zone.

### BRIEF DESCRIPTION OF THE DRAWINGS

Figures 1-2 show process flow diagrams of two embodiments of the invention.

### DETAILED DESCRIPTION OF THE INVENTION

The feedstocks for this invention are an alkylation substrate and an alkylating agent. The alkylation substrate may be essentially any hydrocarbon which is retained as an easily flowable liquid phase material and which may be alkylated via solid catalyst at the conditions employed in the alkylation reactor. The alkylation substrate may be an aromatic hydrocarbon, if the objective is to produce such chemicals as ethylbenzene and cumene or to produce linear alkyl benzenes, which are sulfonated to detergents. Although benzene is the principal aromatic of interest, aromatics such as alkyl-substituted benzenes, condensed ring systems generally, and alkylated derivatives thereof may be used. Examples of such aromatics are toluene, ethylbenzene, propylbenzene, and so forth; xylene, mesitylene, methylethylbenzene, and so on; naphthalene, anthracene, phenanthrene, methyl naphthalene, dimethylnaphthalene, and tetralin. More than one aromatic can be used. If, on the other hand, the objective is to produce motor fuels, then the alkylation substrate may be a paraffinic hydrocarbon, such as a branched paraffin having from 4 to 6 carbon atoms. Suitable paraffinic hydrocarbons are illustrated by 2-methylpropane (commonly called isobutane), 2-methylbutane (or isopentane), 2,3-dimethylbutane, 2-methylpentane, and 3-methylpentane.

The alkylation substrate is alkylated with an alkylating agent. If the objective is to produce chemicals such as ethylbenzene or cumene or to produce motor fuels, then the alkylating agent is typically an olefinic hydrocarbon containing from 2 to about 6 carbon atoms. Examples of such olefins include ethylene, propylene, 1-butene, cis-2-butene, trans-2-butene, and iso-butene. However, if the objective is to produce linear alkyl benzenes, then the alkylating agent can be an olefinic hydrocarbon having from about 2 to about 20 carbon atoms, and usually from about 10 to about 15 carbon atoms. More than one olefin may be used. The alkylating agent may be chosen also from a variety of compounds other than olefins including monohydric alcohols. Suitable alcohols include ethanol and methanol. For instance, methanol is widely described in the literature as being useful in the methylation of benzene and toluene.

The subject process can be performed using any solid, that is, heterogeneous, catalyst which is stable and has the required activity and selectivity for the desired reaction at the conditions needed to maintain liquid phase reactants in the alkylation reactor. In addition, the catalyst must be capable of catalytically alkylating the alkylation substrate with the alkylating agent while also producing a reactor effluent stream that contains not only alkylate but also a hereinafter-described halogen-containing species. Types of catalysts that fulfill this requirement include catalysts that comprise a halide and catalysts that are catalytically promoted by a halide. Individual catalysts within these types are, however, not necessarily equivalent in terms of their catalytic ability to alkylate a given alkylation substrate with a given alkylating agent.

The present invention is applicable to a variety of hydrocarbon alkylation processes. However, the most widely practiced hydrocarbon alkylation process to which the present invention is applicable is motor fuel alkylation. Therefore, the discussion of the invention contained herein will be in reference to its application to a catalytic motor fuel alkylation system. It is not intended that such discussion limit the scope of the invention as set forth in the claims.

A large number of catalysts have been proposed for the production of motor fuel by alkylation including nonzeolitic catalysts and various zeolitic catalysts. Suitable nonzeolitic catalysts include sulfated zirconia and tungstated zirconia. Among suitable zeolitic catalysts, US-A- 4,384,161, for example, describes the use of a large pore zeolite and a Lewis acid. The zeolites referred to include ZSM-4, ZSM-3, the faujasites including zeolite Y, and mordenite. The Lewis acids mentioned in this reference include boron trifluoride and aluminum chloride. A somewhat similar catalyst system comprising a large pore crystalline molecular sieve such as a pillared silicate or an aluminophosphate or silicoaluminophosphate together with a gaseous Lewis acid is disclosed in US-A- 4,935,577. US-A-5,157,200 describes a catalyst comprising a crystalline transition alumina, preferably eta or gamma alumina, which has been treated with a Lewis acid under anhydrous conditions. US-A- 5,157,196 describes a slurried solid catalyst, with the preferred catalyst being an acid washed silica, which has been treated with antimony pentafluoride. Both of these last two references describe a number of prior art heterogeneous paraffin alkylation catalysts.

A preferred paraffin alkylation catalyst comprises a refractory inorganic oxide impregnated with a monovalent cation, especially an alkali metal cation or an alkaline earth metal cation, and whose bound surface hydroxyl groups have been at least partially reacted with a Friedel-Crafts metal halide. Analogs of these catalysts without the metal cations are described in US-A- 2,999,074 and 3,318,820, which describe preparation techniques that can be applied to the preferred catalysts. The preferred refractory oxide is alumina having a surface area greater than 50 m²/g, but the use of other oxides including titania, zirconia, silica, boria, and aluminum phosphate is contemplated. The preferred catalyst also contains a metal component active for olefin hydrogenation deposited on the inorganic oxide prior to reaction of the bound surface hydroxyl groups with the metal halides. This metal may be chosen from the group consisting of nickel, platinum, palladium, and ruthenium with the first three of these metals being preferred. The catalyst contains one or more monovalent metal or alkaline earth metal cations selected from the group consisting of lithium, sodium, potassium, cesium, silver, copper, beryllium, magnesium, calcium, and barium. After the deposition of these metals and the controlled calcination of the composite, the composite is reacted with a Friedel-Crafts metal halide. The metal may be aluminum, zirconium, tin, tantalum, gallium, antimony, or boron. Suitable halides are the fluorides, chlorides, and bromides.

Silicalites have been described as useful alkylation catalysts for the production of monoalkylbenzenes in US-A- 4,489,214 (J. R. Butler et al.) and as useful in methylating toluene to produce paraxylene in US-A- 4,444,989 (F. E. Herkes). The use of ZSM-5 zeolites in aromatic alkylation is described in US-A-3,751,506. ZSM-5 zeolites that have been treated with one or more compounds or elements to improve their selectivity for paraselective alkylation of aromatic hydrocarbons are described in US-A- 4,420,418. The use of zeolite L, zeolite omega, and zeolite beta as alkylation catalysts for the selective alkylation of benzene is described in US-A- 4,301,316. The use of a number of natural and synthetic zeolites including clinoptilolite and zeolite Y as alkylation catalysts is described in US-A- 3,251,897.

The catalyst may be in the form of any suitable shape and size that results in a solid catalyst which flows readily in both dry and wet states and which is readily fluidized at the moderate liquid flow rates employed in a transport reactor such as a riser-reactor. The catalyst can therefore be present as small irregular particles or as uniformly shaped particles. It is preferred that the catalyst is present as "microspheres" having an average diameter of from about 0.1 to about 2.0 mm and more preferably less than about 1.0 mm.

The catalyst is generally employed in a transport reactor. Transport reactors are commonly used in hydrocarbon processing. In a transport reactor, the catalyst bed as a whole moves. Thus, a transport reactor can be contrasted with a fixed bed catalytic reactor and with an ebulliated bed catalytic reactor. In a fixed bed reactor the catalyst particles do not move, and in an ebullated bed reactor the catalyst particles are suspended in a fluid but the settling velocity of the catalyst particles balances the fluid upflow velocity so that the catalyst bed as a whole does not move. Although it is generally the case that the direction of catalyst flow through a transport reactor is upward, the direction may also be downward, horizontal, a direction that is intermediate between vertical and horizontal, or a combination of these directions.

When the direction of catalyst flow through a transport reactor is upward, the transport reactor is often called a riser-reactor. Riser-reactors are commonly used in hydrocarbon processing, such as fluidized catalytic cracking and more recently in fluidized solid bed motor fuel alkylation. In a common arrangement, a fluid hydrocarbon reactant engages a solid hydrocarbon conversion catalyst at the bottom of a riser-reactor and transports the catalyst in a fluidized state up the riser-reactor. A stream of catalyst and hydrocarbon products, by-products, and unreacted reactants if any discharges from the top of the riser-reactor into a separation zone. The hydrocarbons and the catalyst disengage in the separation zone, with the hydrocarbons being withdrawn overhead for recovery and the catalyst dropping by gravity to the bottom of the separation zone. Despite some deactivation that may have occurred to the catalyst in the riser-reactor, some of the catalyst that collects at the bottom of the separation zone usually has enough residual activity that it can be reused in the riser-reactor without first being withdrawn from the separation zone for regeneration. Such still-active catalyst is recirculated through a recirculation conduit from the bottom of the separation zone to the bottom of the riser-reactor, where the catalyst contacts reactants again.

Several methods are used for controlling the introduction of reactants and for controlling the recirculation of catalyst to the bottom of the riser-reactor. For example, US-A- 5,489,732 (Zhang et al.) shows isoparaffins and olefins introduced into the bottom of the riser-reactor, and catalyst flowing through a single recirculation conduit to the bottom of the riser-reactor under control means such as slide valves, other types of valves, lock hoppers, fluid flow control (reverse flow of liquid), screw conveyors, and L-valves. This patent also teaches that one reactant, isobutane, can also be introduced into the recirculation conduit for flushing by-product hydrogen from the recirculating catalyst.

Suitable operating conditions for the riser-reactor include a temperature of from about -50 to about 100° C (-58 to 212°F), preferably from about 0 to about 40°C (32 to 104°F), and a pressure as required to maintain the hydrocarbons present as a liquid. A moderate pressure in the general range of from about 1380 to about 4830 kPa(g) (200 to 700 psi(g)) is preferred with from about 3100 to about 4140 kPa(g) (450 to 600 psi(g)) being highly preferred. The weight ratio of catalyst per olefin in the riser-reactor is generally from about 3 to about 10. The liquid residence time in the riser-reactor is in the general range of from about 60 to about 150 seconds, and the catalyst residence time is in the general range of from about 90 to about 300 seconds. The riser-reactor is preferably designed and operated in a manner intended to promote plug flow (minimal backmixing) of the reactants, products, and catalyst within the riser-reactor. However, the liquid must flow upward faster than the catalyst in order to transport it.

It is generally preferred that the riser-reactor is operated with an excess of the substrate hydrocarbon compared to the alkylating agent. That is, it is preferred to operate with a ratio of the substrate paraffinic or aromatic hydrocarbon to an alkylating agent olefin at the reactor or tube entrance greater than 1:1, and preferably from about 5:1 to about 20:1 or higher as measured by the flow rates into the riser-reactor. It is highly preferred to operate with an abundance of isoparaffin compared to alkylating agent in a motor fuel alkylation process. Specifically, it is preferred that the molar ratio of isoparaffin to olefin being charged to the riser-reactor is greater than 2:1 and more preferably greater than 8:1. Ratios of 10:1 or higher can be employed for motor fuel alkylation, but ratios of about 100:1 or higher are generally considered to be uneconomical. Injection of the olefin at a number of points along the flow path of the hydrocarbon through the riser-reactor may be employed to maintain a higher average paraffin to olefin ratio, and preferably three injection points, in addition to the olefin injection at the bottom of the riser-reactor, are used. So, there are generally four or more olefin injection points along the length of the riser-reactor.

The alkylation reaction effluent generally also contains the desired product of alkylation (alkylate), byproducts of side reactions, and unreacted feedstock. For example, in a process for the production of motor fuel by alkylating butenes with isobutane, alkylation reaction effluent typically comprises hydrocarbons having from 1 to 12 carbon atoms, including methane, ethane, propane, propene, butanes, butenes, pentanes, pentenes, hexanes, heptanes, octanes, nonanes, decanes, undecanes, and dodecanes. The alkylation reaction effluent generally comprises a halogen-containing species also, and the halogen-containing species is present in a concentration of generally greater than about 250 wt-ppm halogen, and usually from about 1000 to about 10000 wt-ppm halogen, based on the weight of the alkylation reaction effluent.

The halogen-containing species in the alkylation reaction effluent can be any halogen-containing species that is not readily separable from molecular hydrogen by fractionation. "Not readily separable from molecular hydrogen by fractionation" means that the volatility difference between molecular hydrogen and the halogen-containing species is so small that, at the column operating pressure, either an undesirably low temperature (i.e., less than 0°C (32°F) would be required to produce reflux, or an undesirably high temperature (i.e., more than 260°C (500°F) would be required to produce boil-up, or a very large number of stages of fractionation (i.e., more than 20 theoretical stages) would be required to achieve the desired separation between molecular hydrogen and the halogen-containing species. Examples of halogen-containing species include molecular fluorine, molecular chlorine, molecular bromine, hydrogen fluoride, hydrogen chloride, and hydrogen bromide.

The catalyst that is employed in the alkylation reaction zone is withdrawn and subject to a mild regeneration, a severe regeneration, or both. When the withdrawn catalyst is subjected to both a mild and a severe regeneration, the mild and severe regeneration zones may be in parallel, so that one portion of the catalyst undergoes mild regeneration, another portion of the catalyst undergoes severe regeneration, and after having each been regenerated the two portions are returned to the alkylation reaction zone. Alternatively, the mild and severe regeneration zones may be in series, so that the portion of the catalyst that undergoes mild regeneration thereafter undergoes severe regeneration.

Mild regeneration comprises contacting the catalyst in a regeneration zone with a liquid-phase hydrocarbon, which is preferably the feed alkylation substrate, such as isobutane. The average residence time of catalyst particles in the liquid-phase hydrocarbon regeneration zone is preferably from about 2 to 20 minutes. Although the liquid-phase or mild regeneration zone may be performed in a separate vessel or conduit that is in communication with the reaction zone, preferably the mild regeneration occurs in the same vessel that contains the reaction zone, provided that the mild regeneration zone is separated from the reaction zone by suitable partitions or baffles. The temperature and pressure conditions in this regeneration zone are similar to those at the reaction zone outlet. The catalyst is treated with molecular hydrogen at a partial pressure between 6.89 to 13790 kPa(g) (1 and2000 psi(g)). The temperature at which the catalyst is treated with molecular hydrogen varies between about 10 to 300°C (50 and about 572°F). Regeneration time depends inversely with temperature. Consequently, higher temps are favored if a shorter regeneration time is desirable, and for this reason temperatures even higher than 300°C (572°F) may be used, although these are not generally recommended. However, other factors favor low temperature regeneration. Regeneration at alkylation process conditions is most desirable in order to eliminate the costs of heating and cooling, and to make regeneration operationally simpler and easier. While regeneration may be done in the temperature range between about 10 to 200°C (50 and about 392°F), the temperature range of from about 38 to 66°C (100 to about 150°F) is preferred. A regeneration time on the order of about 20 minutes suffices to effect restoration of catalyst activity.

The mild regeneration effluent typically comprises molecular hydrogen and hydrocarbon that are introduced into the mild regeneration zone to effect mild regeneration. In a motor fuel alkylation process, the introduced hydrocarbon is typically isobutane. The mild regeneration effluent generally contains more than 0.5 mol-% hydrogen, but since the mild regeneration also generally employs an introduced hydrocarbon, the mild regeneration effluent will usually contain not more than 10 mol-% hydrogen, and commonly not more than 5 mol-% hydrogen. The concentration in the mild regeneration effluent of the hydrocarbon introduced to effect mild regeneration, which is usually the alkylation substrate (e.g., isobutane), will generally be from about 60 mol% to about 90 mol-%. The balance of the regeneration effluent comprises compounds that are removed from the catalyst during mild regeneration. These compounds can comprise any of the hydrocarbons that are present in the alkylation reaction zone, including the alkylation substrate and the product alkylate, and heavy compounds, and the concentration of each of these compounds relative to each other in the mild regeneration effluent is approximately the same as that in the alkylation reaction effluent. The concentration in the mild regeneration effluent of alkylate product is generally less than 5 mol-%, and that of heavy compounds is generally less than 1 mol-%.

Severe regeneration comprises contacting the catalyst either with a vapor-phase gas stream at a relatively high temperature or with a liquid-phase or mixed liquid-vapor phase at a relatively low temperature. The zone in which this severe regeneration step is performed is operated in a manner that provides a longer residence time for the catalyst particles than that provided by the mild regeneration step. The average residence time of a catalyst particle should be at least 30 minutes and can reach about 12 to 24 hours. When regenerating with a vapor-phase gas stream, such as a vapor-phase hydrogen-rich gas stream, the temperature is in the range of generally from about 80 to 500°C (176 to about 932°F), and preferably from 100 to 250°C (212 to 482°F). The presence of some isobutane in this gas stream is desirable to increase the heat capacity of the gas and therefore increase catalyst heat up rates. The longer residence time that is required for this regeneration step allows the high temperature gas that is charged to the regeneration zone to vaporize liquid that flows into the severe regeneration zone.

The severe regeneration effluent typically comprises molecular hydrogen and hydrocarbon, if any, introduced into the severe regeneration zone to effect severe regeneration. In a motor fuel alkylation process, the introduced hydrocarbon is typically the alkylation substrate, which is usually isobutane. While the severe regeneration effluent generally contains greater than 0.5 mol-% hydrogen, the upper limit on the concentration of molecular hydrogen in the severe regeneration effluent depends on whether a hydrocarbon is introduced with molecular hydrogen in order to perform the severe regeneration. When molecular hydrogen is introduced without also introducing hydrocarbon for severe regeneration, the severe regeneration effluent will generally contain more than 80 mol-%, and commonly more than 90 mol-%, hydrogen. In this case, the balance of the regeneration effluent comprises compounds that are removed from the catalyst during severe regeneration. These compounds can comprise any of the hydrocarbons that are present in the alkylation reaction zone, including the alkylation substrate and the product alkylate, and heavy compounds, and the concentration of each of these compounds relative to each other in the severe regeneration effluent is approximately the same as that in the alkylation reaction effluent. The concentration in the severe regeneration effluent of alkylation substrate (e.g., isobutane) relative to that of alkylate product or to that of heavy compounds may be increased by flushing the catalyst with alkylation substrate prior to severe regeneration. Accordingly, the severe alkylation effluent generally contains the alkylation substrate, either because excess alkylation substrate is present on the catalyst when the catalyst was withdrawn from the alkylation reaction zone, or because alkylation substrate was used to flush the catalyst prior to severe regeneration. The concentration in the severe regeneration effluent of alkylate product is generally less than 5 mol-%, and that of heavy compounds is generally less than 1 mol-%.

When a hydrocarbon is introduced along with molecular hydrogen in order to perform severe regeneration, the severe regeneration effluent will generally contain less than 10 mol-% hydrogen, and commonly less than 5 mol-% hydrogen. In this case, severe regeneration occurs in a liquid or mixed liquid-vapor phase and the temperature is in the range of from about 66 to 149°C (150 to about 300°F). In this case also, the concentration in the severe regeneration effluent of the hydrocarbon used during severe regeneration, which is usually the alkylation substrate (e.g., isobutane), will generally be from about 70 mol-% to about 90 mol-%. As in the case of severe regeneration where molecular hydrogen is introduced without also introducing hydrocarbon, when hydrocarbon is introduced along with molecular hydrogen the concentration in the severe regeneration effluent of alkylate product is generally less than 5 mol-%, and that of heavy compounds is generally less than 1 mol-%.

In either mild or severe regeneration, the substrate-containing stream, if any, that is used is usually a distillate cut from a fractionation column and consequently contains other light paraffins besides isobutane. Thus, the mild or severe regeneration effluents may contain other hydrocarbons besides isobutane, such as methane, ethane, propane, normal butane, and pentanes. However, even when these other light hydrocarbons are present, the total concentration of these other light hydrocarbons in the mild or severe regeneration effluent is generally less than 25 mol-% of the concentration of the isobutane.

The mild regeneration effluent, the severe regeneration effluent, or both, passes to the hydrogen fractionation zone. The hydrogen fractionation zone removes and recycles molecular hydrogen in the regeneration effluent of a solid catalyst alkylation process, thereby avoiding passing the molecular hydrogen to the alkylation reactor.

The hydrogen fractionation zone may comprise a rectification section, a stripping section, or both. Where the hydrogen fractionation zone comprises only a rectification section the hydrogen fractionation zone is referred to herein as a hydrogen rectifier, and where the hydrogen fractionation zone comprises only a stripping section the hydrogen fractionation section is referred to herein as a hydrogen stripper. Preferably, the hydrogen fractionation zone is a hydrogen stripper. The hydrogen fractionation zone contains generally from 5 to 50, and preferably from 10 to 20, trays. These numbers of trays are computed based on the assumption that each tray has an efficiency of 30%. If any of the actual trays has an efficiency greater than that assumed, then the numbers of required trays may be lower, and similarly if any of the actual trays has an efficiency that is less than that assumed, then the numbers of required trays may be higher. The hydrogen fractionation zone, including its trays and other internals, may be constructed from carbon steel.

In the case where the hydrogen fractionation zone is a hydrogen stripper, the operating conditions of the hydrogen stripper include a bottoms temperature of generally from about 93 to 138°C (200°F to about 280°F) and preferably from about 110 to 121 °C (230°F to about 250°F), an overhead temperature of generally from about 66 to 121°C (150°F to about 250°F) and preferably from about 82 to 104°C (180°F to about 220°F), and an overhead pressure of generally from about 2758 to 3103 kPa(g) (400 psi(g) to about 450 psi(g)). The overhead stream of the hydrogen fractionation zone contains generally from about 10 to about 60 mol-% hydrogen. Generally greater than about 60%, preferably greater than about 90%, and more preferably greater than about 95% of the moles of molecular hydrogen that enter the hydrogen fractionation zone exit in the overhead stream. While removing molecular hydrogen from the regeneration effluent, the hydrogen fractionation zone should not strip too large of an amount of heavy compounds into the hydrogen fractionation zone overhead stream. Accordingly, the overhead stream of the hydrogen fractionation zone contains generally less than about 0.1 mol-%, and preferably less than about 0.01 mol-%, heavy compounds.

The hydrogen fractionation zone should ensure that molecular hydrogen is removed so that the bottom stream of the hydrogen fractionation zone has a concentration of molecular hydrogen of generally less than about 1.0 mol-% hydrogen, and preferably less than about 0.1 mol-% hydrogen. The remainder of the hydrogen fractionation zone bottom stream comprises liquid hydrocarbons, such as alkylate product and heavy compounds. The quantity of molecular hydrogen that is present in the bottom stream is such that, if all of the molecular hydrogen in the bottom stream ultimately passed to the alkylation reaction zone, then, even if all of that molecular hydrogen reacted with alkylating agent that is being charged to the alkylation reaction zone, then preferably less than 1%, and more preferably less than 0.5%, of the total alkylating agent charged to the alkylation reaction zone would be rendered ineffective by such reaction. An example of rendering the alkylating agent ineffective to react with the alkylation substrate is converting an olefinic alkylating agent feedstock to a paraffinic compound by reacting molecular hydrogen with carbon-carbon double bonds of the olefinic alkylating agent to form carbon-carbon single bonds. The alkylating agent is rendered ineffective to react with the alkylation substrate when the result of the reaction with molecular hydrogen produces a compound which does not have a carbon-carbon double bond. In another embodiment, the ratio of the moles of molecular hydrogen in the net bottom stream withdrawn from the hydrogen stripper to the moles of monoolefinic alkylating agent passed to the alkylation reaction zone is generally less than 0.01, and preferably less than 0.005 In yet another embodiment, the moles of molecular hydrogen in the net hydrogen stripper bottom stream is generally less than 1%, and preferably less than 0.5%, of the moles of carbon-carbon double bonds in the olefinic alkylating agent passed to the alkylation reaction zone.

The hydrogen fractionation zone may also remove hydrogen halide from the regeneration effluent. Removal of hydrogen halide is not a necessary function in the hydrogen fractionation zone. Hydrogen halide in the regeneration effluent may exit the hydrogen fractionation zone either via the overhead stream or via the bottom stream. Any hydrogen halide that exits in the overhead stream is recycled to the regeneration zone and maintains the halide content of the catalyst that exits the regeneration zone. Hydrogen halide that exits in the bottom stream and is then recovered in the alkylate fractionation zone overhead stream is recycled to the alkylation reaction zone. It is believed that even though reintroduction of hydrogen halide to the alkylation reaction zone may react some of the olefinic alkylating agent with hydrogen halide to form a halogenated paraffin, the halogenated paraffin may nevertheless react with the alkylation substrate to produce alkylate. By contrast, any olefinic alkylating agent that reacts with molecular hydrogen is believed to form an unhalogenated paraffin that does not readily react with the alkylation substrate to produce alkylate.

Nevertheless, the hydrogen fractionation zone is generally operated to achieve a desired split of the hydrogen halide between the overhead stream and the bottom stream, to optimize the presence of hydrogen halide in the regeneration zone and in the reaction zone. Generally from 30 to 60%, and preferably from 40 to 50%, of the moles of hydrogen halide that enter the hydrogen fractionation zone exit in the overhead stream, and generally from 40 to 70%, and preferably 50 to 60%, of the entering moles of hydrogen halide exit in the bottom stream. The overhead stream of the hydrogen fractionation zone contains generally 0.1 to 10 mol-% hydrogen halide, and preferably 0.5 to 5 mol-% hydrogen halide. The bottom stream of the hydrogen fractionation zone contains generally from 0.01 to 1 mol-% hydrogen halide, and preferably from 0.05 to 0.5 mol-% hydrogen halide. Compared to the liquid-phase stream produced by a vapor-liquid separator, this invention's hydrogen fractionation zone produces a bottom stream that has a lower concentration of molecular hydrogen, given the same split of the entering hydrogen chloride between the vapor-phase and liquid-phase streams in the vapor-liquid separator and the overhead and bottom streams in the hydrogen fractionation zone.

The alkylation process of this invention has at least one regeneration zone and the effluent from at least one of the regeneration zones passes to at least one hydrogen fractionation zone. Multiple regeneration zones may use a separate or common hydrogen fractionation zone. Preferably all the effluents from all of the regeneration zones pass to a single, common hydrogen fractionation zone to minimize capital costs.

The alkylation reaction effluent and the bottom stream of the hydrogen fractionation zone pass to the alkylate fractionation zone. The alkylate fractionation zone removes the alkylation substrate and halogen-containing species from the alkylate, so that they can be recycled to the alkylation reaction zone.

The alkylate fractionation zone may be a fractionation column having a rectification section without a stripping section, a stripping section without a rectification section, or preferably both rectification and stripping sections. The rectification section of the alkylate fractionation zone contains generally from 1 to 20 trays, preferably from 3 to 10 trays, and more preferably from 1 to 5 trays, presuming as above that the trays have an efficiency of about 60%. The stripping section of the alkylate fractionation zone contains generally from 1 to 150, preferably from 30 to 100, and more preferably from 50 to 75 trays, based on the assumption that these trays in the stripping section have an efficiency of from about 75 to about 100%.

The operating conditions of the alkylate fractionation column include a bottoms temperature of generally from 163 to 218°C (325°F to about 425°F) and preferably from 166 to 193°C (330°F to about 380°F), an overhead temperature of generally from 49 to 71 °C (120°F to about 160°F) and preferably from 49 to 66°C (120°F to about 150°F), and an overhead pressure of generally from 827 to 1379 kPa(g) (120 psi(g) to about 200 psi(g)). The overhead stream of the alkylate fractionation column has a concentration of molecular hydrogen of generally less than about 500 mol-ppm hydrogen, and preferably less than about 100 mol-ppm hydrogen. Preferably the quantity of molecular hydrogen present in the overhead stream would if fully reacted with alkylating agent entering the alkylation zone, render less than 1%, and more preferably less than 0.5%, of the total alkylating agent ineffective to react with the alkylation substrate by such reaction. In another embodiment, the molar ratio of molecular hydrogen in the net overhead stream from the alkylate fractionation column to monoolefinic alkylating agent passed to the alkylation reaction zone is generally less than 0.01, and preferably less than 0.005. In yet another embodiment, the moles of molecular hydrogen in the net alkylate fractionation column overhead stream is generally less than 1%, and preferably less than 0.5%, of the moles of carbon-carbon double bonds in the olefinic alkylating agent passed to the alkylation reaction zone.

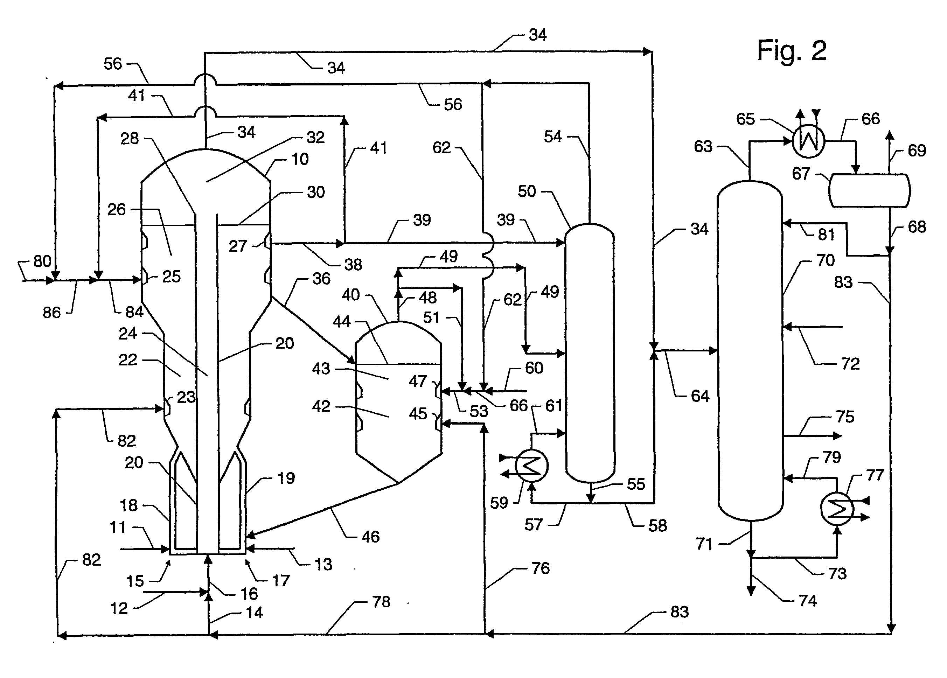

Figures 1 and 2 show simplified embodiments of the process of the subject invention. The description that follows is written in terms of isobutane as the alkylation substrate and a mixture of butene isomers as the alkylation agent, but the choice of these particular reactants for the following description is also not intended to limit the scope of the invention as set forth in the claims.

Referring now to Figure 1, a liquid phase stream comprising isobutane flows through line 14, and a liquid phase stream comprising butene isomers enters the process through line 12. The isobutane combines with the butene isomers, and the combined stream moves through line 16 to the bottom of riser-reactor 20 in vessel 10. The liquid injection lifts the contents of the riser-reactor 20 including solid catalyst from multiple transfer lines 18 and 19. Lines 18 and 19 deliver streams of catalyst to the bottom of riser-reactor 20 at rates controlled by L-valves 15 and 17, respectively. Liquid phase isobutane flows into L-valves 15 and 17 through lines 11 and 13, respectively, at rates sufficient to cause a continuous liquid and solid flow through transfer lines 18 and 19. Catalyst admixes with the entering combined reactant stream in line 16 and catalyzes the reaction of butenes with isobutane to form C₈ product hydrocarbons. The reaction of the olefins and the isobutane takes place in cylindrical space 24 defined by riser-reactor 20. The reaction products, the residual isobutane, and the now used catalyst exit from the top 28 of riser-reactor 20 and enter a large volume cylindrical separation chamber 32.

Low liquid velocities within separation chamber 32 allow the liquids in the riser-reactor to separate from the solid particulate catalyst. The reactor effluent, taken by line 34 recovers liquids that exit the riser-reactor 20 comprising alkylate, isobutane, and other hydrocarbons. The solid catalyst particles settle downward and form catalyst particle bed 26 having an upper limit or surface 30. The catalyst particles in bed 26 will be fluidized to at least the point of minimum fluidization. Bed 26 is preferably a dense fluidized bed or a moving packed bed, and more preferably a moving packed bed. A liquid phase stream, which comprises isobutane, dissolved hydrogen, and a mild regeneration effluent from line 41, passes into vessel 10 through line 84. A conventional fluid flow distributor, such as annular baffle 25, introduces the fluid in line 84 into vessel 10 in order to uniformly distribute the fluid through bed 26. Annular baffles are disclosed in US-A- No. 4,662,081 (Greenwood); 4,665,632 (Greenwood); and 5,397,458 (Micklich et al.). Contact with hydrogen-saturated isobutane subjects catalyst in bed 26 to a mild regeneration procedure, which removes heavy compounds from the catalyst.

Fluid from line 84 together with isobutane from line 82 gradually travel upward through bed 26 in vessel 10. Line 38 withdraws the fluid from bed 26 at a higher point then liquid entering from line 84, but below upper limit or surface 30 of bed 26. Conventional fluid flow collectors, such as annular baffle 27, and particle distributors may withdraw upward flowing fluid from downflowing catalyst particles. Suitable fluid collectors and particle distributors include any device that provides a low velocity disengaging space to allow liquids to be drawn upward while permitting solid particulate catalyst to settle downward. Line 38 removes the collected liquid phase hydrocarbons, any entrained hydrogen, and heavy compounds, from vessel 10 as a mild regeneration effluent. All of the mild regeneration effluent may be passed through lines 39 and 52 to hydrogen stripper 50, but is preferably divided into two portions. Each portion is preferably an aliquot portion of the mild regeneration effluent. As used herein, the term "aliquot portion" of a stream means a portion of the stream that has essentially the same composition as the stream. Figure 1 shows an aliquot portion of the mild regeneration effluent recycled to bed 26 through line 41 and line 86.

Most of the used catalyst from bed 26 passes downward through vessel 10 to catalyst particle bed 22. The catalyst in bed 22 flows downward countercurrent to up-flowing isobutane from line 82, which has a concentration of molecular hydrogen of less than 500 mol-ppm hydrogen. This contacting or washing of the catalyst with isobutane having a concentration of molecular hydrogen of less than 500 mol-ppm hydrogen prevents or at least minimizes the entrance of molecular hydrogen, via transfer lines 18 and 19, into riser-reactor 20, where the molecular hydrogen could saturate olefins added by line 12. If the alkylation catalyst does not promote the hydrogenation of the olefins, this washing procedure may be eliminated. Catalyst particle bed 22 may be fluidized to any extent above the point of minimum fluidization or may be a dense fluidized bed, but preferably bed 22 is a moving packed bed. Annular baffle 23 distributes isobutane from line 82 uniformly across bed 22.

Line 36 withdraws a second and smaller portion of the catalyst present in the catalyst particle bed 26. This smaller stream of catalyst comprising solid catalyst particles and liquid phase hydrocarbons passes to an external regenerator 40 having a bed of catalyst 42. Line 36 discharges catalyst into external regenerator 40 at or above a surface 44 of catalyst beds 43. External regenerator 40 retains catalyst for some average time determined by the catalyst transfer rate. This second catalyst stream may have a uniform flow rate or a variable flow rate to facilitate batch regeneration.

A liquid phase stream, which comprises isobutane, dissolved hydrogen, and a portion of the severe regeneration effluent recycled from line 51, passes into external regenerator 40 through line 53. Annular baffle 47 uniformly distributes the fluid from line 53 through bed 43 at or near the point of fluid introduction. Heating of stream in line 53 to a higher temperature than the stream in line 84 causes a more intense regeneration and a higher temperature in bed 43 than bed 26. The temperature in bed 43 is, however, insufficient to vaporize the liquid phase hydrocarbons that enter external regenerator 40 through lines 53, and bed 43 operates in the liquid phase.

Line 48 withdraws the fluid entering external regenerator 40 through line 53 and isobutane entering through line 76 after its gradual upward movement through bed 43. The point of withdrawal of line 48 is usually above the surface 44 of bed 43 and at or near the highest point in external regenerator 40. Line 48 contains liquid phase hydrocarbons along with any entrained hydrogen, as well as heavy compounds which have been removed from the catalyst that has undergone severe regeneration as the severe regeneration effluent. The severe regeneration effluent may pass in its entirety through lines 49 and 52 to hydrogen stripper 50, but is preferably divided into two portions and more preferably two aliquot portions. Thus, line 51 recycles an aliquot portion of the severe regeneration effluent and combines it with the stream flowing in line 66.

The severely regenerated catalyst retained in catalyst particle bed 43 passes downward through external regenerator 40 to catalyst particle bed 42. In bed 42, the catalyst flows downward countercurrent to isobutane having a concentration of molecular hydrogen of less than 500 mol-ppm hydrogen, which enters external regenerator 40 through line 76. Contacting or washing of the catalyst with isobutane from line 76 prevents or at least minimizes the entrance of molecular hydrogen into riser-reactor 20 via line 46 and any resulting saturation of olefins. If the alkylation catalyst does not promote the hydrogenation of the olefins, this washing may be eliminated. Bed 42 operates in the liquid phase. The entering isobutane liquid having a concentration of molecular hydrogen of less than 500 mol-ppm hydrogen is distributed uniformly across bed 42 by an annular baffle 45. The isobutane and the purged hydrogen, gradually exit the top of bed 42, and ultimately external regenerator 40 through bed 43.

Beds 42 and 43 undergo fluidization to at least the point of minimum fluidization to maintain a dense fluidized or moving packed beds. In the case of batch operation of external regenerator 40, beds 42 and 43 operate as fixed or packed bed during washing and severe regeneration.

Line 46 removes severely regenerated catalyst from external regenerator 40 at a rate that preferably approximates the rate at which catalyst enters external regenerator 40 but that may fluctuate over short periods. A catalyst cooler located below bed 42, in external regenerator 40, or at a point along line 46 may cool the catalyst to a temperature below 38°C (100°F). The severely regenerated catalyst in line 46 commingles with mildly regenerated catalyst flowing through transfer line 19.

The circulation of the catalyst through external regenerator 40 may require the catalyst to be heated and cooled. The utility requirements of the process also require removal of the heat of reaction of the alkylation reaction. The operation of the products recovery section of the process can integrate these activities. For instance, a cooler can return the heat from the severly regenerated catalyst to the product recovery section. Alternatively, such a cooler may return heat to external regenerator 40 to heat the catalyst undergoing severe regeneration.

Aliquot portions of the severe regeneration effluent in line 49 and the mild regeneration effluent in line 39 flow as a combined stream through line 52 to hydrogen stripper 50. The hydrogen stripper 50 typically contains a vapor-liquid contacting medium such as trays or packing, and the combined stream usually enters hydrogen stripper 50 at an elevation above the upper limit or surface of the contacting medium.

The hydrogen stripper 50 strips molecular hydrogen from liquid isobutane and liquid heavy compounds as these liquids descend through hydrogen stripper 50. Hydrogen chloride, which is less volatile than molecular hydrogen at the operating conditions of hydrogen stripper 50, is also stripped from the descending liquids. Line 54 withdraws molecular hydrogen and hydrogen chloride from hydrogen stripper 50 in a stripper overhead stream. The stripper overhead stream comprises a gaseous or vapor stream of molecular hydrogen, hydrogen chloride, and isobutane, but has low concentrations of alkylate and heavy compounds.

The stripper overhead stream can be recycled to bed 26, bed 43, or both. A portion, preferably an aliquot portion, of the stripper overhead stream in line 54 passes through line 56 and combines with makeup molecular hydrogen entering the process via line 80 to form a combined stream in line 86. The combined stream in line 86 in turn combines with the recycled portion of the mild regeneration effluent in line 41 and thereby forms the stream in line 84 which enters bed 26. Similarly, another aliquot portion of the stripper overhead stream in line 54 passes through line 62, combines with makeup molecular hydrogen entering the process via line 60, thereby forming a combined stream in line 66. That combined stream in turn mixes with a recycled portion of the severe regeneration effluent in line 51 and thereby forms the stream in line 53 which enters bed 43.

A liquid phase bottom stream exits hydrogen stripper 50 through line 55. The hydrogen stripper bottom stream comprises isobutane and hydrocarbons that are heavier than isobutane, including pentanes, alkylate, and heavy compounds. The hydrogen stripper 50 strips at least a portion of the molecular hydrogen from the descending liquids, so that the molecular hydrogen content of the hydrogen stripper bottom stream in line 55 is generally less than that of the combined stream entering the hydrogen stripper 50 via line 52. One portion of the stripper bottom stream may pass through line 57 and an optional reboiler 59, and line 61. Reboiler 59 provides any necessary heat required for stripping molecular hydrogen in hydrogen stripper 50.

Another portion of the stripper bottom stream flows through line 58, combines with reactor effluent flowing through line 34, and the combined stream enters alkylate product recovery column 70. Thus, the combined stream in line 64 comprises alkylate, heavy compounds, isobutane, hydrogen chloride, and trace if any hydrogen. The alkylate product recovery column 70 separates isobutane from normal butane and heavier liquid hydrocarbons including the product alkylate, and is commonly referred to as an "isostripper." Makeup field butanes, comprising isobutane and normal butane, enter isostripper 70 through line 72. Isostripper 70 operates as a fractionation column with a stripping section and a rectification section, and typically contains approximately 60 to 80 trays, based on a tray efficiency of 60-90 %. Line 75 withdraws a sidecut stream of normal butane from a sidecut tray located between the feed tray and the bottom of isostripper 70. Line 63 withdraws an overhead stream comprising isobutane and hydrogen chloride from the top of isostripper 70. Typically, condenser 65 condenses most of the overhead and then passes it through line 66 to receiver 69, for vapor-liquid phase separation. Line 69 withdraws an overhead vapor stream comprising hydrogen chloride, hydrocarbons lighter than isobutane, hydrogen, if any, and any other uncondensable components from receiver 67. Line 68 withdraws an overhead liquid stream comprising isobutane with a concentration of molecular hydrogen of less than 500 mol-ppm hydrogen that generally also comprises hydrogen chloride. Line 70 refluxes an aliquot portion of the overhead liquid stream to the isostripper 70, and the remaining aliquot portion flows through line 83. Lines 76 and 78 further divide the aliquot portion of line 83 into aliquot portions that enter external regenerator 40 and riser-reactor 20 and bed 22 via lines 14 and 82.

An isostripper bottoms stream comprising alkylate is withdrawn from isostripper 70 through line 71. Line 74 recovers the net product alkylate from the portion of the isostripper bottom stream that passes through line 73, reboiler 77, and line 79.

In a variation on the flow arrangement of Figure 1 but not shown in Figure 1, the stripper overhead stream in line 54 first passes to a condenser which condenses the isobutane. The condenser outlet stream, which is a mixture of molecular hydrogen, hydrogen chloride, and condensed isobutane, then passes to an overhead receiver, which separates the mixture into vapor and liquid phases. The vapor phase, comprising mostly molecular hydrogen and hydrogen chloride, recycles to beds 26 and/or 43, and the isobutane liquid phase may be passed to beds 26 and/or 42, to riser-reactor 20, or to another location in the process where a stream of liquid isobutane may be useful, such as reflux to the hydrogen stripper itself. This variation permits permits recycle of the stripped isobutane independently of the stripped molecular hydrogen and hydrogen chloride and provides also a very low molecular hydrogen content in the hydrogen stripper bottoms stream if desired. In this case, the hydrogen stripper is operated at severe stripping conditions, thereby stripping from the hydrogen stripper bottoms and into the hydrogen stripper overhead not only more molecular hydrogen but also more isobutane. Using a condenser/receiver system in the hydrogen stripper overhead thus allows the stripped isobutane to be separated from the stripped molecular hydrogen and hydrogen chloride.

Figure 2 shows another embodiment of the invention wherein catalyst in the severe regeneration zone of external regenerator 40 contacts a vapor phase regeneration stream. Corresponding items in Figures 1 and 2 have the same reference number. In Figure 2, heater(s) which are not shown vaporize the isobutane that passes to external regenerator via lines 53 and 76. A vapor phase effluent exits the severe regeneration zone via line 48. Mild regeneration effluent in line 39 and severe regeneration effluent in line 49 flow separately to hydrogen stripper 50. The liquid phase mild regeneration effluent portion enters the upper portion of hydrogen stripper 50 and the vapor phase severe regeneration effluent portion enters at a lower location in hydrogen stripper 50. The feed point of the mild regeneration effluent portion is preferably above the first tray and the feed point of the severe regeneration effluent portion is below half the trays.

### EXAMPLE

In accordance with Figure 1, an olefinic and a paraffinic feed having the compositions shown in Table 1 pass to an alkylation reaction zone employing a solid alkylation catalyst and produce a reaction effluent having the composition shown in Table 2. The solid alkylation catalyst is regenerated in a mild regeneration zone and in a severe regeneration zone, and effluents having the compositions shown in Table 2 are withdrawn from these two zones. The regeneration effluents pass to a hydrogen stripper, which produces an overhead stream and a bottom stream having the compositions shown in Table 2. 99 percent of the molecular hydrogen and from 40 to 60 percent of the hydrogen chloride that enters the hydrogen stripper with the regeneration effluents exits in the hydrogen stripper overhead stream, with the remainder of the entering molecular hydrogen and hydrogen chloride exiting with the hydrogen stripper bottom stream. The reactor effluent and the hydrogen stripper bottom stream are combined into a combined feed that has the composition shown in Table 2 and is fed to an isostripper. Because the combined feed to the isostripper is in part formed from the hydrogen stripper bottom stream, which contains only 0.02 mol-% hydrogen, rather than from the mild regeneration effluent (2.3 mol-% hydrogen) or the severe regeneration effluent (2.8 mol-% hydrogen), the combined feed to the isostripper contains a low concentration of molecular hydrogen (only 0.006 mol-%). Accordingly, the isostripper need not be designed or operated in a manner so that large quantities of molecular hydrogen must be separated from the entering hydrocarbons in order to prevent the molecular hydrogen from being recycled to the alkylation reaction zone.

**Table 1**

| Composition of Feeds - mol % | | |
|---|---|---|
| | Olefinic Feed | Paraffinic Feed |
| Molecular hydrogen | - | 0.027 |
| Hydrogen chloride | - | 0.2 |
| Methane | - | 0.2 |
| Ethane | - | 0.6 |
| Propane | 0.1 | 8.4 |
| Propene | 0.2 | - |
| Butanes | 34.4 | 89.7 |
| Butenes | 44.8 | - |
| Pentanes | 12.3 | 0.8 |
| Pentenes | 8.0 | - |
| Hexanes and heavier hydrocarbons | 0.2 | 0.1 |
| Total | 100.0 | 100.0 |

**Table 2**

| Composition of Streams -- mol % | | | | | | |
|---|---|---|---|---|---|---|
| Stream | Reactor Effluent | Mild Regeneration Effluent | Severe Regeneration Effluent | Hydrogen Stripper Overhead | Hydrogen Stripper Bottom | Combined Feed to Isostripper |
| Line Number in Figure 1 | 34 | 39 | 49 | 54 | 55 | 64 |
| Hydrogen | - | 2.3 | 2.8 | 34.7 | 0.020 | 0.006 |
| Hydrogen Chloride | 0.1 | 0.3 | 0.3 | 1.9 | 0.2 | 0.1 |
| Hydrocarbons | 99.9 | 97.4 | 96.9 | 63.4 | 99.8 | 99.9 |
| Total | 100.0 | 100.0 | 100.0 | 100.0 | 100.0 | 100.0 |

## Claims

1. An alkylation process comprising:
a) passing a first feed stream comprising a paraffinic alkylation substrate and a second feed stream comprising an olefinic alkylating agent to an alkylation reaction zone operated at alkylation conditions selected to react the paraffinic alkylation substrate and the olefinic alkylating agent in the presence of a solid catalyst to produce alkylate, the alkylation conditions being sufficient to deposit heavy compounds on the solid catalyst in the alkylation reaction zone, and withdrawing from the alkylation reaction zone an alkylation reaction effluent comprising the alkylate and the paraffinic alkylation substrate;
b) withdrawing a first catalyst stream comprising solid catalyst having heavy compounds deposited thereon from the alkylation reaction zone, passing at least a portion of the first catalyst stream to a first regeneration zone, contacting the solid catalyst having heavy compounds deposited thereon with molecular hydrogen in the first regeneration zone at first regeneration conditions selected to remove at least a portion of the heavy compounds from the solid catalyst having heavy compounds deposited thereon and to at least partially regenerate the solid catalyst having heavy compounds deposited thereon;
c) withdrawing a second catalyst stream comprising at least partially regenerated solid catalyst from the first regeneration zone, and passing at least a portion of the second catalyst stream to the alkylation reaction zone;
d) withdrawing a first regeneration effluent comprising molecular hydrogen and the heavy compounds from the first regeneration zone, passing at least a portion of the first regeneration effluent to a hydrogen fractionation zone, and recovering from the hydrogen fractionation zone a hydrogen-enriched stream having a first concentration of molecular hydrogen and a hydrogen-depleted stream comprising the heavy compounds and having a second concentration of molecular hydrogen that is less than the first concentration of molecular hydrogen;
e) passing at least a portion of the hydrogen-enriched stream to the first regeneration zone;
f) passing at least a portion of the alkylation reaction effluent and at least a portion of the hydrogen-depleted stream to an alkylate fractionation zone, and withdrawing from the alkylate fractionation zone a recycle stream comprising the paraffinic alkylation substrate;
g) forming the first feed stream from at least a portion of the recycle stream; and
h) recovering the alkylate from the alkylate fractionation zone.

2. The process of Claim 1 further **characterized in that** the solid catalyst comprises a halide, the alkylation reaction effluent comprises a halogen-containing species, and the recycle stream comprises the halogen-containing species.

3. The process of Claim 1 further **characterized in that** the first regeneration effluent has a concentration of molecular hydrogen of greater than about 0.5 mol-% hydrogen.

4. The process of Claim 1 further **characterized in that** the ratio of the moles of molecular hydrogen in the first feed stream to the moles of olefinic alkylating agent in the second feed stream is less than 0.01.

5. The process of Claim 1 further **characterized in that** the moles of molecular hydrogen in the first feed stream is less than 1 % of the moles of carbon-carbon double bonds in the olefinic alkylating agent in the second feed stream.

6. The process of Claim 1 further **characterized in that** the hydrogen-depleted stream has a concentration of molecular hydrogen of less than 1.0 mol-%, and **in that** the recycle stream has a concentration of molecular hydrogen of less than 500 mol-ppm hydrogen.

7. The process of Claim 1 further **characterized in that** the first regeneration effluent comprises a halogen-containing species and that from 30 to 60% of the halogen-containing species in the at least a portion of the first regeneration effluent is recovered from the hydrogen fractionation zone in the hydrogen-enriched stream.

8. The process of Claim 1 further **characterized in that** the first regeneration conditions comprise at least a partial liquid phase.

9. The process of Claim 1 wherein the halogen is fluoride, chloride, or bromide and the halogen-containing species is hydrogen fluoride, hydrogen chloride, and hydrogen bromide.

10. The process of Claim 1 wherein the paraffinic alkylation substrate comprises a paraffin selected from the group consisting of 2-methylpropane, 2-methylbutane, 2,3-dimethylbutane, 2-methylpentane, and 3-methylpentane and the olefinic alkylating agent comprises an olefin selected from the group consisting of ethylene, propylene, 1-butene, cis-2-butene, trans-2-butene, and iso-butene.

11. The process of Claim 1 further **characterized in that** a third catalyst stream comprising the solid catalyst having heavy compounds deposited thereon is withdrawn from the alkylation reaction zone, at least a portion of the third catalyst stream passes to a second regeneration zone, molecular hydrogen contacts the solid catalyst having heavy compounds deposited thereon in the second regeneration zone at second regeneration conditions to remove at least a portion of the heavy compound deposits from the solid catalyst having heavy compounds deposited thereon and to at least partially regenerate the solid catalyst having heavy compounds deposited thereon, a fourth catalyst stream comprising at least partially regenerated solid catalyst is withdrawn from the second regeneration zone, at least a portion of the fourth catalyst stream passes to the alkylation reaction zone, a second regeneration effluent comprising molecular hydrogen and heavy compounds is withdrawn from the second regeneration zone, and at least a portion of the second regeneration effluent passes to the hydrogen fractionation zone.

12. The process of Claim 11 further **characterized in that** the first regeneration conditions comprise a first regeneration temperature and the second regeneration conditions comprise a second regeneration temperature that is greater than the first regeneration temperature.

## Patentansprüche

1. Alkylierungsverfahren, bei dem man:
a) einen ersten Einsatzstoffstrom, der ein paraffinisches Alkylierungssubstrat umfaßt, und einen zweiten Einsatzstoffstrom, der ein olefinisches Alkylierungsmittel umfaßt, einer Alkylierungszone zuführt, die unter Alkylierungsbedingungen betrieben wird, die so gewählt sind, daß das paraffinische Alkylierungssubstrat und das olefinische Alkylierungsmittel in Gegenwart eines festen Katalysators zu Alkylat reagieren, wobei die Alkylierungsbedingungen zur Abscheidung von schweren Verbindungen auf dem festen Katalysator in der Alkylierungsreaktionszone ausreichen, und aus der Alkylierungsreaktionszone einen Alkylierungsreaktionsaustragsstrom, der das Alkylat und das paraffinische Alkylierungssubstrat umfaßt, abzieht;
b) aus der Alkylierungsreaktionszone einen ersten Katalysatorstrom, der festen Katalysator mit darauf abgeschiedenen schweren Verbindungen umfaßt, abzieht, mindestens einen Teil des ersten Katalysatorstroms einer ersten Regenerationszone zuführt, den festen Katalysator mit darauf abgeschiedenen schweren Verbindungen in der ersten Regenerationszone unter ersten Regenerationsbedingungen, die so gewählt sind, daß mindestens ein Teil der schweren Verbindungen von dem festen Katalysator mit darauf abgeschiedenen schweren Verbindungen entfernt wird und der feste Katalysator mit darauf abgeschiedenen schweren Verbindungen mindestens teilweise regeneriert wird, mit molekularem Wasserstoff in Berührung bringt;
c) aus der ersten Regenerationszone einen zweiten Katalysatorstrom, der mindestens teilweise regenerierten festen Katalysator umfaßt, abzieht und mindestens einen Teil des zweiten Katalysatorstroms der Alkylierungsreaktionszone zuführt;
d) aus der ersten Regenerationszone einen ersten Regenerationsaustragsstrom, der molekularen Wasserstoff und die schweren Verbindungen umfaßt, abzieht, mindestens einen Teil des ersten Regenerationsaustragsstroms einer Wasserstofffraktionierungszone zuführt und aus der Wasserstofffraktionierungszone einen wasserstoffangereicherten Strom, der eine erste Konzentration von molekularem Wasserstoff aufweist, und einen wasserstoffabgereicherten Strom, der die schweren Verbindungen umfaßt und eine zweite Konzentration von molekularem Wasserstoff, die niedriger ist als die erste Konzentration von molekularem Wasserstoff, aufweist, gewinnt;
e) mindestens einen Teil des wasserstoffangereicherten Stroms der ersten Regenerationszone zuführt;
f) mindestens einen Teil des Alkylierungsreaktionsaustragsstroms und mindestens einen Teil des wasserstoffabgereicherten Stroms einer Alkylatfraktionierungszone zuführt und aus der Alkylatfraktionierungszone einen Rückführungsstrom, der das paraffinische Alkylierungssubstrat umfaßt, abzieht;
g) den ersten Einsatzstrom aus mindestens einem Teil des Rückführungsstroms bildet und
h) aus der Alkylatfraktionierungszone das Alkylat gewinnt.

2. Verfahren nach Anspruch 1, ferner **dadurch gekennzeichnet, daß** der feste Katalysator ein Halogenid umfaßt, der Alkylierungsreaktionsaustragsstrom eine halogenhaltige Spezies umfaßt und der Rückführungsstrom die halogenhaltige Spezies umfaßt.

3. Verfahren nach Anspruch 1, ferner **dadurch gekennzeichnet, daß** der erste Regenerationsaustragsstrom eine Konzentration von molekularem Wasserstoff von mehr als etwa 0,5 Mol-% Wasserstoff aufweist.

4. Verfahren nach Anspruch 1, ferner **dadurch gekennzeichnet, daß** das Verhältnis der Mole von molekularem Wasserstoff im ersten Einsatzstoffstrom zu den Molen von olefinischem Alkylierungsmittel im zweiten Einsatzstoffstrom weniger als 0,01 beträgt.

5. Verfahren nach Anspruch 1, ferner **dadurch gekennzeichnet, daß** sich die Mole von molekularem Wasserstoff im ersten Einsatzstoffstrom auf weniger als 1% der Mole von Kohlenstoff-Kohlenstoff-Doppelbindungen im olefinischen Alkylierungsmittel im zweiten Einsatzstoffstrom belaufen.

6. Verfahren nach Anspruch 1, ferner **dadurch gekennzeichnet, daß** der wasserstoffabgereicherte Strom eine Konzentration von molekularem Wasserstoff von weniger als 1,0 Mol-% aufweist und der Rückführungsstrom eine Konzentration von molekularem Wasserstoff von weniger als 500 Mol-ppm Wasserstoff aufweist.

7. Verfahren nach Anspruch 1, ferner **dadurch gekennzeichnet, daß** der erste Regenerationsaustragsstrom eine halogenhaltige Spezies umfaßt und 30 bis 60% der halogenhaltigen Spezies in dem mindestens einen Teil des ersten Regenerationsaustragsstroms aus der Wasserstofffraktionierungszone in dem wasserstoffangereicherten Strom gewonnen werden.

8. Verfahren nach Anspruch 1, ferner **dadurch gekennzeichnet, daß** die ersten Regenerationsbedingungen mindestens eine teilweise flüssige Phase umfassen.

9. Verfahren nach Anspruch 1, bei dem es sich bei dem Halogen um Fluorid, Chlorid oder Bromid handelt und es sich bei der halogenhaltigen Spezies um Fluorwasserstoff, Chlorwasserstoff und Bromwasserstoff handelt.

10. Verfahren nach Anspruch 1, bei dem das paraffinische Alkylierungssubstrat ein Paraffin aus der Gruppe bestehend aus 2-Methylpropan, 2-Methylbutan, 2,3-Dimethylbutan, 2-Methylpentan und 3-Methylpentan umfaßt und das olefinische Alkylierungsmittel ein Olefin aus der Gruppe bestehend aus Ethylen, Propylen, 1-Buten, cis-2-Buten, trans-2-Buten und Isobuten umfaßt.

11. Verfahren nach Anspruch 1, ferner **dadurch gekennzeichnet, daß** man aus der Alkylierungsreaktionszone einen dritten Katalysatorstrom, der den festen Katalysator mit darauf abgeschiedenen schweren Verbindungen umfaßt, abzieht, mindestens einen Teil des dritten Katalysatorstroms einer zweiten Regenerationszone zuführt, den festen Katalysator mit darauf abgeschiedenen schweren Verbindungen in der zweiten Regenerationszone unter zweiten Regenerationsbedingungen, unter denen mindestens ein Teil der Abscheidungen schwerer Verbindungen von dem festen Katalysator mit darauf abgeschiedenen schweren Verbindungen entfernt wird und der feste Katalysator mit darauf abgeschiedenen schweren Verbindungen mindestens teilweise regeneriert wird, mit molekularem Wasserstoff in Berührung bringt, aus der zweiten Regenerationszone einen vierten Katalysatorstrom, der mindestens teilweise regenerierten festen Katalysator umfaßt, abzieht und mindestens einen Teil des vierten Katalysatorstroms der Alkylierungsreaktionszone zuführt, aus der zweiten Regenerationszone einen zweiten Regenerationsaustragsstrom, der molekularen Wasserstoff und schwere Verbindungen umfaßt, abzieht und mindestens einen Teil des zweiten Regenerationsaustragsstroms der Wasserstofffraktionierungszone zuführt.

12. Verfahren nach Anspruch 11, ferner **dadurch gekennzeichnet, daß** die ersten Regenerationsbedingungen eine erste Regenerationstemperatur umfassen und die zweiten Regenerationsbedingungen eine zweite Regenerationstemperatur, die größer ist als die erste Regenerationstemperatur, umfassen.

## Revendications

1. Procédé d'alkylation comprenant :
a) le passage d'un premier courant d'alimentation comprenant un substrat d'alkylation paraffinique et d'un second courant d'alimentation comprenant un agent d'alkylation oléfinique dans une zone de réaction d'alkylation fonctionnant dans des conditions d'alkylation choisies pour faire réagir le substrat d'alkylation paraffinique et l'agent d'alkylation oléfinique en présence d'un catalyseur solide pour produire un alkylat, les conditions d'alkylation étant suffisantes pour déposer des composés lourds sur le catalyseur solide dans la zone de réaction d'alkylation, et le soutirage de la zone de réaction d'alkylation d'un effluent de réaction d'alkylation comprenant l'alkylat et le substrat d'alkylation paraffinique ;
b) le soutirage de la zone de réaction d'alkylation d'un premier courant de catalyseur comprenant du catalyseur solide ayant des composés lourds déposés sur celui-ci, le passage d'au moins une partie du premier courant de catalyseur dans une première zone de régénération, la mise en contact du catalyseur solide ayant des composés lourds déposés sur celui-ci avec de l'hydrogène moléculaire dans la première zone de régénération dans des premières conditions de régénération choisies pour enlever au moins une partie des composés lourds du catalyseur solide ayant des composés lourds déposés sur celui-ci et pour régénérer au moins partiellement le catalyseur solide ayant des composés lourds déposés sur celui-ci ;
c) le soutirage de la première zone de régénération d'un deuxième courant de catalyseur comprenant du catalyseur solide au moins partiellement régénéré et le passage d'au moins une partie du deuxième courant de catalyseur dans la zone de réaction d'alkylation ;
d) le soutirage de la première zone de régénération d'un premier effluent de régénération comprenant de l'hydrogène moléculaire et les composés lourds, le passage d'au moins une partie du premier effluent de régénération dans une zone de fractionnement de l'hydrogène et la récupération à partir de la zone de fractionnement de l'hydrogène d'un courant enrichi en hydrogène ayant une première concentration en hydrogène moléculaire et d'un courant appauvri en hydrogène comprenant les composés lourds et ayant une seconde concentration en hydrogène moléculaire qui est inférieure à la première concentration en hydrogène moléculaire ;
e) le passage d'au moins une partie du courant enrichi en hydrogène dans la première zone de régénération ;
f) le passage d'au moins une partie de l'effluent de réaction d'alkylation et d'au moins une partie du courant appauvri en hydrogène dans une zone de fractionnement de l'alkylat et le soutirage de la zone de fractionnement de l'alkylat d'un courant de recyclage comprenant le substrat d'alkylation paraffinique ;
g) la formation du premier courant d'alimentation à partir d'au moins une partie du courant de recyclage ; et
h) la récupération de l'alkylat à partir de la zone de fractionnement de l'alkylat.

2. Procédé de la revendication 1 **caractérisé en outre en ce que** le catalyseur solide comprend un halogénure, l'effluent de réaction d'alkylation comprend une espèce halogénée et le courant de recyclage comprend l'espèce halogénée.

3. Procédé de la revendication 1 **caractérisé en outre en ce que** le premier effluent de régénération a une concentration en hydrogène moléculaire supérieure à environ 0,5 % en mole d'hydrogène.

4. Procédé de la revendication 1 **caractérisé en outre en ce que** le rapport du nombre de moles d'hydrogène moléculaire dans le premier courant d'alimentation sur le nombre de moles d'agent d'alkylation oléfinique dans le second courant d'alimentation est inférieur à 0,01.

5. Procédé de la revendication 1 **caractérisé en outre en ce que** le nombre de moles d'hydrogène moléculaire dans le premier courant d'alimentation est inférieur à 1 % du nombre de moles de doubles liaisons carbone-carbone dans l'agent d'alkylation oléfinique dans le second courant d'alimentation.

6. Procédé de la revendication 1 **caractérisé en outre en ce que** le courant appauvri en hydrogène a une concentration en hydrogène moléculaire inférieure à 1,0 % en mole et **en ce que** le courant de recyclage a une concentration en hydrogène moléculaire inférieure à 500 ppm en mole d'hydrogène.

7. Procédé de la revendication 1 **caractérisé en outre en ce que** le premier effluent de régénération comprend une espèce halogénée et **en ce que** de 30 à 60 % de l'espèce halogénée dans l'au moins une partie du premier effluent de régénération est récupérée à partir de la zone de fractionnement de l'hydrogène dans le courant enrichi en hydrogène.

8. Procédé de la revendication 1 **caractérisé en outre en ce que** les premières conditions de régénération comprennent au moins une phase liquide partielle.

9. Procédé de la revendication 1 dans lequel l'halogène est le fluorure, le chlorure ou le bromure et l'espèce halogénée est le fluorure d'hydrogène, le chlorure d'hydrogène et le bromure d'hydrogène.

10. Procédé de la revendication 1 dans lequel le substrat d'alkylation paraffinique comprend une paraffine choisie dans le groupe constitué par le 2-méthylpropane, le 2-méthylbutane, le 2,3-diméthylbutane, le 2-méthylpentane et le 3-méthylpentane et l'agent d'alkylation oléfinique comprend une oléfine choisie dans le groupe constitué par l'éthylène, le propylène, le 1-butène, le *cis*-2-butène, le *trans*-2-butène et l'isobutène.

11. Procédé de la revendication 1 **caractérisé en outre en ce qu'**un troisième courant de catalyseur comprenant le catalyseur solide ayant des composés lourds déposés sur celui-ci est soutiré de la zone de réaction d'alkylation, au moins une partie du troisième courant de catalyseur passe dans une seconde zone de régénération, de l'hydrogène moléculaire est mis en contact avec le catalyseur solide ayant des composés lourds déposés sur celui-ci dans la seconde zone de régénération dans des secondes conditions de régénération pour enlever au moins une partie des dépôts de composés lourds du catalyseur solide ayant des composés lourds déposés sur celui-ci et pour régénérer au moins partiellement le catalyseur solide ayant des composés lourds déposés sur celui-ci, un quatrième courant de catalyseur comprenant du catalyseur solide au moins partiellement régénéré est soutiré de la seconde zone de régénération, au moins une partie du quatrième courant de catalyseur passe dans la zone de réaction d'alkylation, un second effluent de régénération comprenant de l'hydrogène moléculaire et des composés lourds est soutiré de la seconde zone de régénération et au moins une partie du second effluent de régénération passe dans la zone de fractionnement de l'hydrogène.

12. Procédé de la revendication 11 **caractérisé en outre en ce que** les premières conditions de régénération comprennent une première température de régénération et les secondes conditions de régénération comprennent une seconde température de régénération qui est supérieure à la première température de régénération.
